# EUROPEAN PATENT SPECIFICATION

(11) **EP 2 808 328 B1**
(45) Date of publication and mention of the grant of the patent: **20.04.2016**
(21) Application number: 14169956.1
(22) Date of filing: 27.05.2014
(51) Int. Cl.: C07D 487/22

(54) **Pentaphyrins useful in the biomedical field**
Pentaphyrine zur biomedizinischen Anwendungen
Dérivés de pentaphyrins destinés à une utilisation biomédicale

(30) Priority: 27.05.2013 IT UD20130075
(43) Date of publication of application: 03.12.2014
(73) Proprietor: Universita' Degli Studi di Udine, 33100 Udine (IT)
(72) Inventor: Comuzzi, Clara, 33019 Tricesimo (UD) (IT); Goi, Daniele, 33100 Udine (IT)
(74) Representative: Petraz, Gilberto Luigi

(56) References cited:
- EP-A1- 1 316 436
- ALONSO ET AL.: "Conformational Control in [22]- and [24]Pentaphyrins(1.1.1.1.1) by Meso Substituents and their N-Fusion Reaction", J. ORG. CHEM., vol. 78, 3 April 2013 (2013-04-03), pages 4419-4431, XP002715236,
- KRIVOKAPIC ET AL.: "Contracted and Expanded meso-Alkynyl Porphyrinoids: from Triphyrin to Hexaphyrin", J. ORG. CHEM., vol. 68, 2003, pages 1089-1096, XP002715237,
- JI-YOUNG SHIN ET AL.: "N-Fused Pentaphyrin", ANGEW. CHEM. INT. ED., vol. 40, no. 3, 2001, pages 619-621, XP002715238,
- JI-YOUNG SHIN ET AL.: "Meso-Aryl-Substituted Expanded Porphyrins", J. AM. CHEM. SOC., vol. 123, 2001, pages 7190-7191, XP002715239,

## Description

### FIELD OF THE INVENTION

Embodiments of the present disclosure relate to novel pentaphyrins, which can be used for instance in the biomedical field, or in cation or anion binding applications or for manufacturing near infrared materials.

In particular, embodiments described herein can be used for acting as photosensitizers for photodynamic therapy (PDT).

Embodiments described herein also relate to methods for producing novel pentaphyrins.

### BACKGROUND OF THE INVENTION

Expanded porphyrins are porphyrogenic macrocycles where five or more pyrroles are linked together mainly through direct α, α connections or CH= bridges. In the past decades many macrocycles have been synthesized and characterized allowing the better understanding of fundamental concepts as aromaticity, π conjugation structural dependence and discovering new physical and chemical features expanding the application potential of these molecules [1]. The plethora of fields in which they can be applied spans from biomedical, to cation or anion binding application and lately near infrared materials [2]. Near infrared materials are now attracting a lot of attention as a consequence of their application in sectors such as energy, communication, bio-imaging, sensing and advanced optoelectronics. Near infrared materials can be divided in materials able to absorb light in the NIR region of the electromagnetic spectrum or materials able to emit light in the NIR region. Data concerning NIR features of expanded porphyrin start to be collected showing their enormous potential in this field [3].

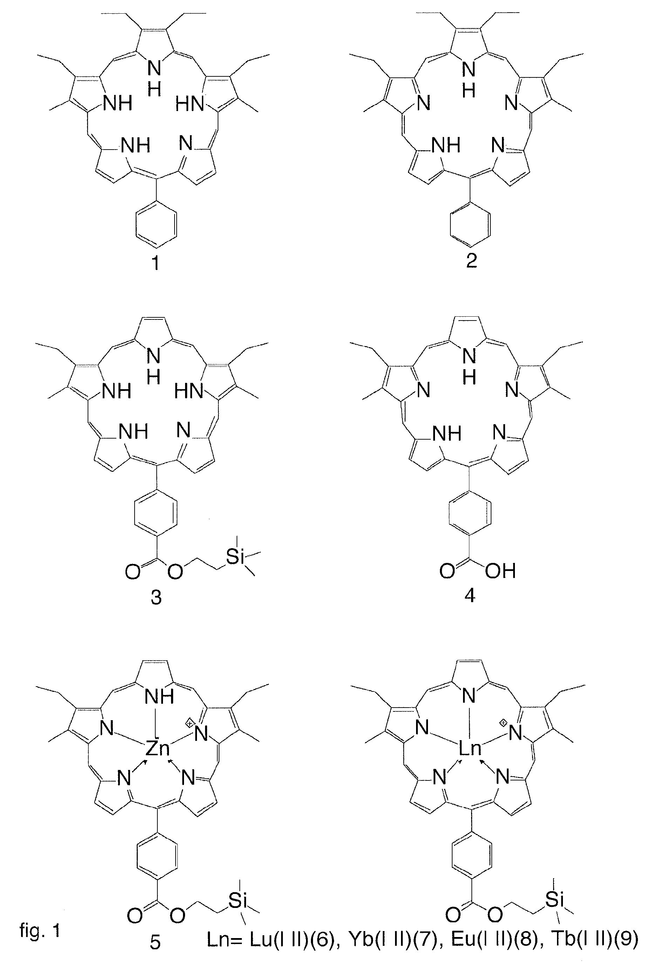

Recently we dedicated part of our research to the synthesis and characterization of [1.1.1.1.1] pentaphyrins, a class of expanded porphyrins made of five pyrrolic rings linked by means of *meso*-like bridges. Our data demonstrated that [1.1.1.1.1] pentaphyrins exist in two different oxidation states, the non aromatic (reduced form) 24 π-electrons iso-pentaphyrins (1 and 3) and the aromatic (oxidized form) 22 π-electrons pentaphyrin (2) (Figure 1) [4]. The stability, accessibility, and also spectroscopic features of the reduced and oxidized forms depend on structural features of the macrocycle framework. For example our recent results show that the oxidation from a 24 π to a 22 π form can be achieved only in conditions, which favour specific conformations of the macrocycle that, in turn, depend on the framework substitution [4b]. In the past years we tested the ability of pentaphyrins to act as photosensitizers for photodynamic therapy (PDT). Photodynamic therapy (PDT) is an emerging cancer treatment modality based on the excitation of a nontoxic photosensitizer with harmless visible light to produce reactive-oxygen species (ROS)[5]. The oxidant conditions realized by the irradiation of the photosensitizer can be exploited also in environmental application to catalyse pollutant degradation and water disinfection [6]. Recently the photoinactivation properties of a novel, expanded porphyrin, namely 20-(4'-carboxyphenyl)-2,13-dimethyl-3,12-diethyl-[22]pentaphyrin (PCox, **4),** were tested in water disinfection, using *S. Aureus* as a Gram-positive bacteria model. Data showed that PCox was effective against S. *Aureus* bacteria at nanomolar concentration [7]. We also demonstrated that the iso-pentaphyrins and pentaphyrins are promoting different types of light activated reactions and that metal ions insertion into iso-pentaphyrins increases dramatically the ROS species production (heavy metal effect) [4c]. Currently, only few attempts to obtain pentaphyrins have been accomplished, and the few examples of [1.1.1.1.1] pentaphyrins isolated and characterized are mostly alkyl substituted compounds at the β position of pyrroles. [8, 9] Some of them are water soluble, [10] but actually a very small number of pentaphyrins with *meso-*substituents are known. [11, 4b, 12]

Giving an excursus of the synthetic approaches present in literature, we found that the first [22] pentaphyrinic macrocycle was obtained by Gossauer *et al.,* through a [3 + 2] Mac-Donald type condensation between a diformyl tripyrrane and αα'-free dipyrromethane.[13] Later, Sessler and co-workers improved the conditions, isolating a *meso*-free α-alkyl-substituted pentaphyrin and reporting the crystal structure of the corresponding uranium complex. [8] If, on the other hand, a modified Rothemund method consisting of a *one-pot* reaction between pyrrole and corresponding benzaldehyde is applied, *meso*-aryl substituted N-fused pentaphyrin containing a tripentacyclic ring can be obtained as demonstrated by Furuta *et al.*[14]. *meso*-Aryl *β*-free macrocycles display interesting structural diversities not observed for the *β*-substituted pentaphyrins, and show unexpected optical properties. N-fusion and N-cofusion phenomena are observable, when *meso*-aryl *β*-free substituted pentaphyrins are present. [14, 15].

Scientific publication Alonso *et al.* [16] discloses conformational control in [22]- and [24]Pentaphyrins (1.1.1.1.1) by meso substituents and their N-fusion reaction obtained by computational methods. No synthesis is disclosed in [16]. According to Alonso *et al.,* the triangular conformation with two stacked pyrrole rings is preferred for fully *meso*-aryl-substituted pentaphyrins irrespective of the oxidation state. This conformation is very prone to undergo a N-fusion reaction. The formation of the N-fused structures from this doubly inverted conformation is thermodynamically spontaneous in both [22] Pentaphyrins and [24] Pentaphyrins. According to Alonso *et al.,* nonfused [24]PP are unstable regardless of the substituents. The computational results provided in Alonso *et al.* support experimental evidence available for *meso*-pentaphyrins(1.1.1.1.1). The results of Alonso *et al.* teach the fully *meso*-aryl-substituted pentaphyrins can exist only as N-fused forms, *i.e.* according to Alonso *et al.* fully *meso-*aryl-substituted pentaphyrins are not available in stable form. According to Alonso *et al.,* the removal of one aryl group prevents the N-fusion reaction, providing stable aromatic nonfused [22]pentaphyrins.

There is therefore a need to improve pentaphyrins, to provide fully *meso-*substituted pentaphyrins, and to provide a method for the production of novel fully *meso*-substituted pentaphyrins, in particular for, but not limited to, use in the biomedical field, or in cation or anion binding applications or for manufacturing near infrared materials, more particularly for acting as photosensitizers for photodynamic therapy (PDT), which overcomes at least one of the drawbacks of the prior art.

### SUMMARY OF THE INVENTION

According to embodiments, novel fully *meso*-substituted (5, 10, 15, 20, 25) R-[1.1.1.1.1] [22] pentaphyrins or fully *meso*-substituted (5, 10, 15, 20, 25) R-[1.1.1.1.1] [24] pentaphyrins or their coordination complexes are provided, wherein R is a substituent group selected from a group consisting of: penta(4-pyridyl), penta(N-methyl-pyridinium-4-yl) and penta(phenyl).

According to embodiments, the use of fully *meso*-substituted (5, 10, 15, 20, 25) R-[1.1.1.1.1] [22] pentaphyrins and fully *meso*-substituted (5, 10, 15, 20, 25) R-[1.1.1.1.1] [24] pentaphyrins as biomedical material can be provided.

According to embodiments, the use of fully *meso*-substituted (5, 10, 15, 20, 25) R-[1.1.1.1.1] [22] pentaphyrins and fully *meso*-substituted (5, 10, 15, 20, 25) R-[1.1.1.1.1] [24] pentaphyrins in cation or anion binding applications or protic or neutral, such as water or alcohol, molecules binding applications, can be provided. In possible embodiments, since the band at 470 nm corresponding to the pentaphyrins is in the visible light field, use of the fully *meso*-substituted pentaphyrins or their coordination complexes according to the present disclosure as visible field materials can also be envisaged.

According to embodiments, the use of fully *meso*-substituted (5, 10, 15, 20, 25) R-[1.1.1.1.1.] [22] pentaphyrins and fully *meso*-substituted (5, 10, 15, 20, 25) R-[1.1.1.1.1] [24] pentaphyrins as near infrared materials can be provided.

According to embodiments, the use of fully *meso*-substituted (5, 10, 15, 20, 25) R-[1.1.1.1.1] [22] pentaphyrins and fully *meso*-substituted (5, 10, 15, 20, 25) R-[1.1.1.1.1] [24] pentaphyrins as photosensitizers for photodynamic therapy (PDT) can be provided.

According to embodiments, R is select from a group consisting of: penta(4-pyridyl), penta(N-ethyl-pyridium-4yl) and penta(phenyl).

According to embodiments, a method for the production of fully *meso-*substituted pentaphyrins is provided. The method comprises reacting precursors comprising *meso*-substituted dipyrromethane and *meso*-substituted tripyrromethane and adding a suitable catalyst component and an oxidant component.

These and other features, aspects and advantages of the present disclosure will become better understood with reference to the following description, the drawings and appended claims. The drawings, which are incorporated in and constitute a part of this specification, illustrate embodiments of the present subject matter and, together with the description, serve to explain the principles of the disclosure.

The various aspects and features described in the present disclosure can be applied, individually, wherever possible. These individual aspects, for instance the aspects and features described in the attached dependent claims, can be made subject of divisional patent applications.

It is noted that anything found to be already known during the patenting process is understood not to be claimed and to be the subject of a disclaimer.

### BRIEF DESCRIPTION OF THE DRAWINGS

So that the manner in which the above recited features of the present invention can be understood in detail, a more particular description of the invention, briefly summarized above, may be had by reference to embodiments. The accompanying drawings relate to embodiments of the disclosure and are described in the following:
- Figure 1 shows the structure of the known pentaphyrins already described in [4] and [7];
- Figure 2 shows the general structure of fully *meso*-substituted pentaphyrins according to the present invention;
- Figure 3 shows the structure of possible embodiments of fully *meso*-substituted pentaphyrins according to the present invention;
- Figure 4 shows the structure of the novel fully *meso*-substituted pentaphyrins according to embodiments described herein: 5,10,15,20,25-penta(4-pyridyl)-pentaphyrin (1.1.1.1.1) P5Py **(10),** 5,10,15,20,25-penta(N-methyl-pyridium-4yl)-pentaphyrin (1.1.1.1.1) (5MP5Py) **(11)** and 5,10,15,20,25-penta(phenyl)-pentaphyrin (1.1.1.1.1) (P5Ph) **(12);**
- Figure 5 shows the synthetic routes for novel 5,10,15,20,25-penta(4-pyridyl)-pentaphyrin (1.1.1.1.1) **10** and novel 5,10,15,20,25-penta(N-methyl-pyridium-4yl)-pentaphyrin (1.1.1.1.1) **11;**
- Figure 6 shows the synthetic routes for novel 5,10,15,20,25-penta(phenyl)-pentaphyrin (1.1.1.1.1) **12.**

### DETAILED DESCRIPTION OF THE EMBODIMENTS

Reference will now be made in detail to the various embodiments of the invention, one or more examples of which are described herein. Each example is provided by way of explanation of the invention and is not meant as a limitation of the invention. For example, features illustrated or described as part of one embodiment can be used on or in conjunction with other embodiments to yield yet a further embodiment. It is intended that the present invention includes such modifications and variations.

Embodiments described herein provide novel fully *meso*-substituted (5, 10, 15, 20, 25) R-[1.1.1.1.1] [22] pentaphyrins or their coordination complexes.

Embodiments described herein provide novel fully *meso*-substituted (5, 10, 15, 20, 25) R-[1.1.1.1.1] [24] pentaphyrins or their coordination complexes.

Coordination complexes can be, for instance, metals complexes, such as Ln(III) or Zn(II) or Fe(II/III) complexes.

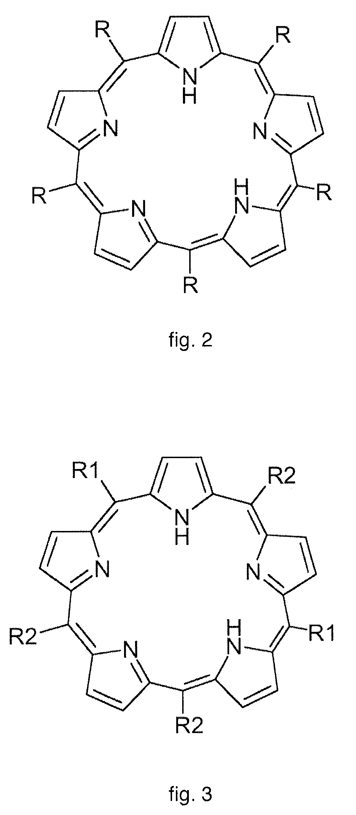

Figure 2 is used to describe embodiments, combinable with all embodiments described herein, of fully *meso*-substituted (5, 10, 15, 20, 25) R-[1.1.1.1.1] [22] pentaphyrins.

Figure 3 is used to describe embodiments according to the present disclosure, in which for example two R1 and three R2 are provided as substituents and R1≠R2.

In possible embodiments, the synthesis and NMR, UV-Vis and mass characterization of fully *meso*-substituted (5, 10, 15, 20, 25) aryl-[1.1.1.1.1] [22] pentaphyrins are hereby described by way of example.

In particular, the synthesis and NMR, UV-Vis and mass characterization of novel pentaphyrins aryl substituted in 5, 10, 15, 20, 25 positions are provided, for instance 5,10,15,20,25-penta(4-pyridyl))-pentaphyrin (1.1.1.1.1) P5Py **(10),** 5,10,15,20,25-penta(N-methyl-pyridium-4yl)-pentaphyrin (1.1.1.1.1) (5MP5Py) **(11)** and 5,10,15,20,25-penta(phenyl)-pentaphyrin (1.1.1.1.1) (P5Ph) **(12).**

5,10,15,20,25-penta(N-methyl-pyridium-4yl)-pentaphyrin (1.1.1.1.1) **11** is believed to be a compound useful in the field of biomedical materials, in particular a compound with a high anti-bacterial activity, i.e. a biocide. By way of example, such property can be used to produce an anti-mould paint.

In possible examples, embodiments described herein provide that the substituent R is not one of C₆F₅ or 2,6-Cl₂-phenyl.

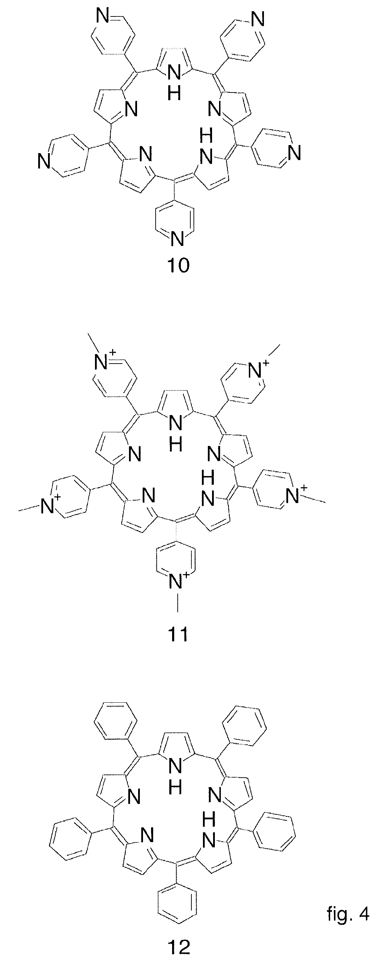

Figure 4 is used to describe embodiments of novel pentaphyrins according to the present disclosure: 5,10,15,20,25-penta(4-pyridyl))-pentaphyrin (1.1.1.1.1) P5Py **(10),** 5,10,15,20,25-penta(N-methyl-pyridium-4yl)-pentaphyrin (1.1.1.1.1) (5MP5Py) **(11)** and 5,10,15,20,25-penta(phenyl)-pentaphyrin (1.1.1.1.1) (PSPh) **(12).**

According to aspects, novel pentaphyrins described herein may be obtained by reacting precursors including *meso-aryl* substituted dipyrromethane and *meso-*aryl substituted tripyrromethane, such as *meso*-(4-pyridyl)dipyrromethane **(13)** and *meso*-(4-pyridyl)tripyrromethane **(14)** or their corresponding phenyl-substituted *meso*-(phenyl)dipyrromethane **(15)** and *meso*-(phenyl) tripyrromethane **(16)** and by addition of a suitable catalyst, for instance trifluoroacetic acid (TFA) catalyst and an oxidant component, for instance 2,3-dichloro-5,6-dicyano-14-benzoquinone (DDQ).

In some embodiments, the catalyst component is trifluoroacetic acid (TFA).

In some embodiments, combinable with all embodiments described herein, the oxidant component is 2,3-dichloro-5,6-dicyano-1,4-benzoquinone (DDQ).

According to embodiments, *meso*-(4-pyridyl)dipyrromethane and *meso-*(4-pyridyl)tripyrromethane are reacted with pyridinecarboxaldehyde in the presence of the suitable catalyst and with subsequent addition of the oxidant component to obtain 5,10,15,20,25-penta(4-pyridyl)-pentaphyrin (1.1.1.1.1).

In some possible implementations, reacting *meso*-(4-pyridyl)dipyrromethane, *meso*-(4-pyridyl)tripyrromethane and pyridinecarboxaldehyde comprises dissolving *meso*-(4-pyridyl)dipyrromethane, *meso*-(4-pyridyl)tripyrromethane and 4-pyridinecarboxaldehyde, adding trifluoroacetic acid (TFA) catalyst to the solution, observing increasingly intense Soret band at 470 nm corresponding to the pentaphyrin and letting the reaction progress until no further gain in Soret band spectral intensity is observed, adding 2,3-dichloro-5,6-dicyano-1,4-benzoquinone (DDQ) oxidant, washing, evaporating and purifying the solution to obtain 5,10,15,20,25-penta(4-pyridyl)-pentaphyrin (1.1.1.1.1).

According to embodiments, the method further comprises dissolving obtained 5,10,15,20,25-penta(4-pyridyl)-pentaphyrin (1.1.1.1.1), adding excess of methyl iodide, stirring the reactant mixture, evaporating the solvent, washing the solid residue to obtain 5,10,15,20,25-penta(N-methyl-pyridium-4yl)-pentaphyrin (1.1.1.1.1).

According to further embodiments, the *meso*-(phenyl)dipyrromethane **(15)** is converted, in the presence of N-benzoylmorpholine and POCl₃, to 1,9-bis(benzoyl)-5-phenyldipyrromethane, then obtained 1,9-bis(benzoyl)-5-phenyldipyrromethane **(17)** is reacted with NaBH₄ to yield the corresponding dicarbinol derivative, i.e. phenyldipyrromethane-dicarbinol **(18)** and then *meso-*(phenyl)tripyrromethane **(16)** and phenyldipyrromethane-dicarbinol **(18)** are reacted with the catalyst component and addition of the oxidant component to give 5,10,15,20,25-pentaphenyl-pentaphyrin (1.1.1.1.1).

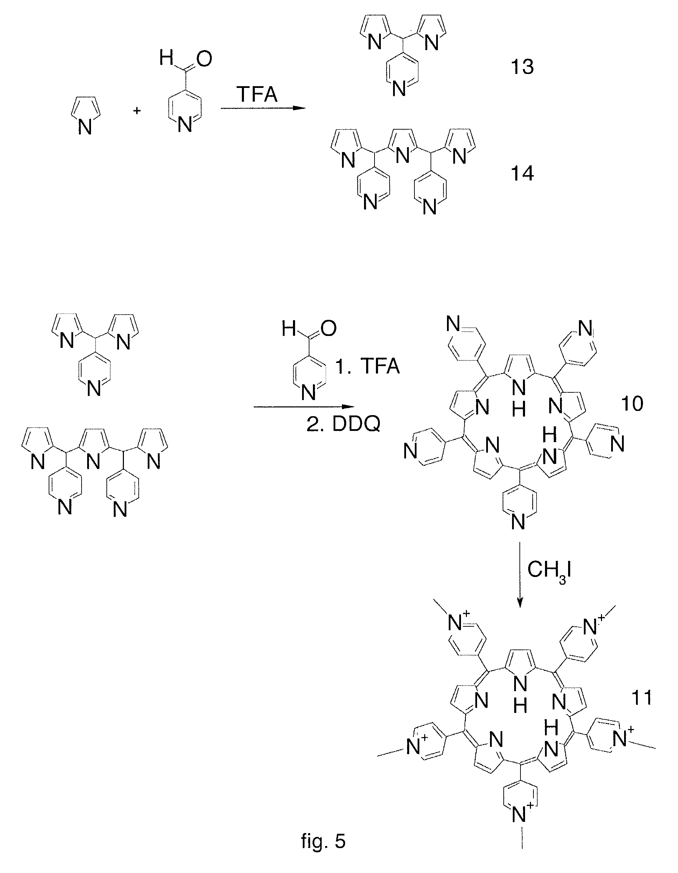

Figure 5 is used to describe exemplifying embodiments of synthetic routes for obtaining novel 5,10,15,20,25-penta(4-pyridyl)-pentaphyrin (1.1.1.1.1) **10** and novel 5,10,15,20,25-penta(N-methyl-pyridium-4yl-pentaphyrin (1.1.1.1.1) **11.** According to embodiments described herein, 5,10,15,20,25-penta(4-pyridyl)-pentaphyrin (1.1.1.1.1) **10** can be obtained by *meso*-(4-pyridyl)dipyrromethane **(13)** and *meso*-(4-pyridyl)tripyrromethane **(14)** reacted with pyridinecarboxylaldheyde, in the presence of a suitable catalyst, such as trifluoroacetic acid (TFA) catalyst.

Referring to Figure 5, synthesis of *meso*-(4-pyridyl)dipyrromethane **(13)** will now be described hereafter according to embodiments described herein.

An amount of 4-pyridinecarboxaldehyde was provided, for example from 0,8 to 1 mL, e.g. 0.9 mL, at a molar concentration of between 8 to 1 mmol, e.g. 9 mmol.

An amount of pyrrole (C₄H₄NH) was provided, for example from 9.0 mL to 9.2 mL, *e.g.* 9.1 mL, at a molar concentration of between 130 mmol to 132 mmol, *e.g.* 131 mmol.

4-pyridinecarboxaldehyde was then stirred with pyrrole. A suitable catalyst, such as trifluoroacetic acid (TFA) catalyst, was added. Stirring can be performed for a defined stirring time, for instance of about 17 hr to 19 hr, *e.g.* 18 hr, at a defined stirring temperature, for example of about 83°C to 87°C, *e.g.* 85°C, under an inert gas atmosphere, *e.g.* nitrogen atmosphere. The stirred mixture was then subjected to drying, purification and recrystallization. For instance, drying can be performed by evaporation to dryness, purification can be performed by alumina flash-chromatography and then recrystallization can be performed (ethyl acetate: hexane) to afford 1.2 g (yield 58%) of the compound **(13)** as yellow pale solid.

*meso*-(4-pyridyl)dipyrromethane **(13)** was thus obtained and then characterized by UV-Vis, ¹H NMR, ¹³C NMR and ESI-MS analysis, with the following results.
UV/vis (CH₂Cl₂): λₘₐₓ (log ε) [nm] = 308 (2.96), 439 (2.25).
¹H NMR (200 MHz, CDC13) δ ppm 8.50 (dd, J=4.7, 1.2 Hz, 2H, 2,6-p_{y}), 8.13 (bs, 2 H, NH), 7.13 (dd, *J*=4.5, 1.4 Hz, 2 H, 3,5-p_{y}), 6.74 (d, *J*=1.38 Hz, 2 H, pyrrole), 6.18 (dd, *J*=2.82, 5.70 Hz, 2 H, pyrrole), 5.90 (m, 2 H), 5.46 (s, 1 H, *CH-meso).*
¹³C NMR (50 MHz, CDCl3) d ppm 151.2, 149.9, 130.6, 123.6, 117.9, 108.6, 107.7, 43.4.
ESI-MS, C₁₄H₁₄N₃ calculated exact mass 224.12 [M+H]⁺, observed 224.19 [M+H]⁺.

Crystal Structure of thus obtained dipyrromethane **(13)** was as follows. C₁₄N₃H₁₃, M_{w} = 223.27. Crystal size: 0.1 x 0.02 x 0.01 mm, Crystal system: Orthorhombic, space group *Pna2₁*(#33), *a* = 5.9611(10), *b* = 12.289(2), *c* = 15.6145(10) Å, *V* = 1143.9(3) Å³, Z= 4, calculated = 1.29 g/cm³.

Referring to Figure 5, synthesis of *meso*-(4-pyridyl)tripyrromethane **(14)** will now be described hereafter according to embodiments described herein.

An amount of 4-pyridinecarboxaldehyde was provided, for example from 1,2 to 1,4 mL, e.g. 1.3 mL, at a molar concentration of between 13 to 15 mmol, *e.g.* 14 mmol.

An amount of pyrrole was provided, for example from 4.7 mL to 4.9 mL, *e.g.* 4.8 mL, at a molar concentration of between 69 mmol to 71 mmol, *e.g.* 70 mmol.

4-pyridinecarboxaldehyde was then stirred with pyrrole. A suitable catalyst, such as TFA catalyst, was added. Stirring can be performed for a defined stirring time, for instance of about 17 hr to 19 hr, *e.g.* 18 hr, at a defined stirring temperature, for example of about 83°C to 87 °C, *e.g.* 85°C, under an inert gas atmosphere, e.g. nitrogen atmosphere.

After stirring, pyrrole was removed at reduced pressure. The crude product, obtained after the removal of pyrrole at reduced pressure, was purified, for instance by alumina flash-chromatography. The purified product was recrystallized. From the recrystallization of the fractions recovered, the following species were isolated: *meso*-(4-pyridyl)tripyrromethane, *i.e*. compound **(14),** 2.3 g (yield 43%).

*meso*-(4-pyridyl)tripyrromethane, *i.e*. compound **(14),** was thus obtained and then characterized by UV-Vis, ¹H -NMR, ¹³C NMR and ESI-MS analysis, with the following results.
UV-Vis (CH₂Cl₂): λₘₐₓ (log ε) [nm] = 320 (2.97), 430 (2.43), 524 (2.25) (sh), 684 (1.53).
¹H NMR (200 MHz, CDCl3) δ ppm 8.42 (dd, *J*=5.8, 1.1 Hz, 4 H, 2,6-p_{y}), 8.28 (bs, 3 H, NH), 7.08 (d, *J*=5.8 Hz, 4 H, 3,5-p_{y}), 6.73 (td, J=2.1, 2.1, 4.0 Hz, 2 H, pyrrole), 6.15 (dd, *J*=2.8, 5.8 Hz, 2 H), 5.86 (m, 2 H, pyrrole), 5.77 (m, 2 H, pyrrole), 5.37 (s, 2 H, CH-*meso*).
¹³C NMR (50 MHz, CDCl₃) δ ppm 146.6, 143.9, 132.6, 129.7, 125.8, 119.9, 110.4, 108.3, 42.2.
ESI-MS, C₁₄H₁₄N₃ calculated exact mass 380.19 [M+H]⁺, observed 380.28 [M+H]⁺.

Referring to Figure 5, synthesis of 5,10,15,20,25-penta(4-pyridyl)-pentaphyrin (1.1.1.1.1) **(10)** will now be described hereafter according to embodiments described herein.

*meso*-(4-pyridyl)dipyrromethane **13,** *meso*-(4-pyridyl)tripyrromethane **14** as obtainable according to embodiments described herein and 4-pyridinecarboxaldehyde were dissolved in a suitable solvent. Then a suitable catalyst, *e.g.* TFA catalyst, was added.

For instance, in a 50-mL round bottom flask *meso*-(4-pyridyl)dipyrromethane **13** (*e.g.* 0.016 g, 0.072 mmol), *meso*-(4-pyridyl)tripyrromethane **14** *(e.g.* 0.027 g, 0.072 mmol) and 4-pyridinecarboxaldehyde *(e.g.* 0.014 mL, 0.143 mmol) were dissolved in 20 mL of anhydrous CH₃CN.

After degassing, the mixture was kept under protected atmosphere, *e.g*. under a nitrogen blanket, and shielded from ambient light, *e.g*. with aluminium foil.

Upon addition of the TFA catalyst (*e.g.* 0.033 mL, 0.430 mmol) the mixture immediately adopted a dark brown hue.

Reaction progress was followed by UV-Vis spectroscopy and ESI-MS spectrometry. The observation of increasingly intense Soret band at 470 nm corresponding to the pentaphyrin was noted.

At the point where (ca. 18 h) no further gain in Soret band spectral intensity was observed, the solution was neutralized, *e.g*. with triethylamine.

Then 2,3-dichloro-5,6-dicyano-1,4-benzoquinone (DDQ) (*e.g.* 0.024 g, 0.108 mmol) was added. DDQ is a highly effective oxidant which can be typically used for instance in a number of chemical transformations, including protecting group removal, aromatization, acetal formation and biaryl construction.

The reaction solution was then left stirring at room temperature for additional time, for example of between 17 hr and 19 hr, *e.g.* 18 hr. The organic solution was then washed, *e.g*. with hot water, and evaporated to give a deep brown/green metallic film. This residue was subjected to purification, *e.g*. by column chromatography on alumina. The column was first eluted with ethylacetate in order to remove tetra(4-pyridyl)porphyrin, followed by ethylacetate containing gradually increasing percentages of methanol (*e.g*. 1 ÷ 10%). The brown-green, pentaphyrin-containing fractions with a characteristic absorption at 473 nm were collected and concentrated under reduced pressure giving for instance 7,2 mg of 5,10,15,20,25-penta(4-pyridyl)-pentaphyrin (1.1.1.1.1) **10** (yield 13%).

5,10,15,20,25-penta(4-pyridyl)-pentaphyrin (1.1.1.1.1), *i*.*e*. compound **10,** was thus obtained and then characterized by UV-Vis, ¹H -NMR and ESI-MS analysis, with the following results.
UV-Vis (CH2Cl2): λₘₐₓ (log ε) [nm] = 352 (4.54), 423 (4.39), 473 (4.52), 562 (4.26) (sh), 702 (402) (br).
¹H NMR (200 MHz, CD₃OD) dppm 8.70 ÷ 8.10 (overlapped signals, 8 H), 7.80 ÷ 7.00 (overlapped signals, 18 H), 6.72 (m, 4H).
ESI-MS, C₅₀H₃₂N₁₀ calculated exact mass 773.30 [M+H]⁺, observed 773.46 [M+H]⁺, 791.43 [M·H2O + H]⁺, 805.44 [M·CH3OH + H]⁺.

Referring to Figure 5, synthesis of 5,10,15,20,25-penta(N-methyl-pyridium-4yl)-pentaphyrin (1.1.1.1.1) **(11)** will now be described hereafter according to embodiments described herein.

The 5,10,15,20,25-penta(4-pyridyl)-pentaphyrin (1.1.1.1.1), *i.e.* compound **10,** obtained (*e.g*. 0.007 g, 0.009 mmol) was dissolved in a suitable solvent, for instance in 15 mL of anhydrous methanol, and a large excess of methyl iodide CH₃I (10 eq) was added. The reactant mixture was stirred, for instance at T = 40°C for 4hr, and monitored with 1H-NMR and ESI-MS. The solvent was then evaporated and the solid *(e.g.* 6.3 mg, yield 80 %) washed with ether. 5,10,15,20,25-penta(N-methyl-pyridium-4yl)-pentaphyrin (1.1.1.1.1) **(11)** was obtained as a deep green solid.

5,10,15,20,25-penta(N-methyl-pyridium-4yl)-pentaphyrin (1.1.1.1.1) **(11)** thus obtained was then characterized by UV-Vis, ¹H -NMR and ESI-MS analysis, with the following results.
UV-Vis (CH3OH): λₘₐₓ (log ε) [nm] = 348 (3.89), 392 (3.95), 462 (3.63), 522 (3.56), 574 (3.42) (sh), 716 (3.04) (freshly made 10⁻⁴M solution).
¹H NMR (200 MHz, CD3OD, T= 60°C) δ ppm 9.17 (d, J=6.6 Hz, 6 H), 9.11 (m, 2 H), 8.95 (d, J=6.4 Hz, 4 H), 8.84 (s, 4 H), 8.45 (d, J=6.7 Hz, 4 H), 8.38 (d, J=6.3 Hz, 6 H), 7.55 (s, 4H), 4.56 (s, 6 H), 3.69 (s, 9 H).
ESI-MS, C₅₅H₄₇N₁₀⁵⁺ calculated exact mass 169.48 [M⁵⁺/5], observed 169.65 [M⁺⁵/5].

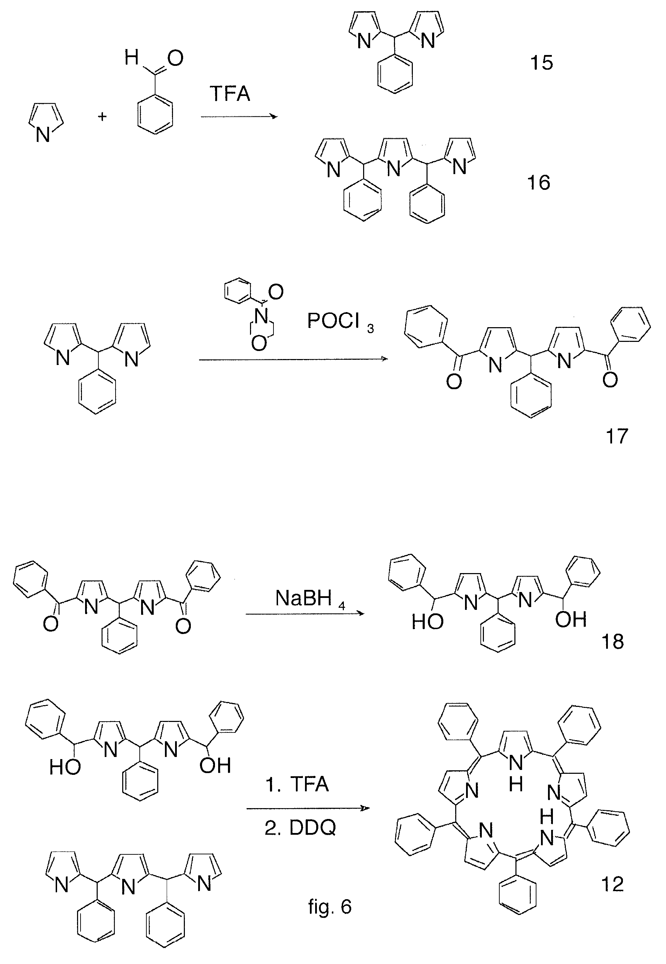

Figure 6 is used to describe embodiments of the synthetic routes for novel 5,10,15,20,25-pentaphenyl-pentaphyrin (1.1.1.1.1) **(12).**

Novel 5,10,15,20,25-pentaphenyl-pentaphyrin (1.1.1.1.1) **12** was obtained starting from *meso*-(phenyl)dipyrromethane **(15)** and *meso-*(phenyl)tripyrromethane **(16).**

The compounds *meso*-(phenyl)dipyrromethane **15** and *meso-*(phenyl)tripyrromethane **16** were obtained and purified as described in literature [4 (a), (b)].

*meso*-(phenyl)dipyrromethane **15** was converted, in the presence of N-benzoylmorpholine and POCl₃, to 1,9-bis(benzoyl)-5-phenyldipyrromethane **(17)** according to literature [17]. 1,9-bis(benzoyl)-5-phenyldipyrromethane **(17)** was reacted with NaBH₄ to yield phenyldipyrromethane-dicarbinol, *i.e*. component **18.** Then *meso*-(phenyl)tripyrromethane **16** and phenyldipyrromethane-dicarbinol **18** were reacted with catalyst(s) to give 5,10,15,20,25-pentaphenyl-pentaphyrin (1.1.1.1.1) **12.**

For instance, 22.2 mg of 1,9-bis(benzoyl)-5-phenyldipyrromethane **17** were dissolved in 2.2 ml of a dry mixture of THF and MeOH (10:1) and placed in a 10 ml round bottom flask under N₂ flux. 0.03783g NaBH₄ were added slowly to give phenyldipyrromethane-dicarbinol **18.** The reaction was followed by TLC (CH₂Cl₂: EtOAc 3:2). The reaction mixture was first poured into a 3 ml of saturated NH₄Cl solution and then 6 ml CH₂Cl₂ were added. The organic phase was separated, washed twice with water, dried over Na₂SO₄ and evaporated. The residue of phenyldipyrromethane-dicarbinol **18** was dissolved in 20 ml of dry CH₃CN and added with 20 mg of *meso*-(phenyl)tripyrromethane **16** under protected atmosphere, e.g. under N₂. The solution was protected by light before injecting 50µl TFA. After 3h stirring at room temperature, 20mg DDQ were poured into the solution and stirred for 1h, neutralized with TEA, passed through an Al₂O₃ pad (eluent CH₂Cl₂) and then through a SiO₂ pad (eluent CH₂Cl₂). The residue was dissolved in CH₂Cl₂ containing 50% suitable catalyst, e.g. TFA and exposed to air for 2h. The dark mixture was evaporated to dryness dissolved in the minimum amount of CH₃CN and precipitated with water. The precipitated was purified on SiO₂ using toluene containing gradually increasing percentages of Ethylacetate (1 ÷ 10%). The purple fraction of pure 5,10,15,20,25-pentaphenyl-pentaphyrin (1.1.1.1.1) **12** P5Ph was collected when toluene containing 10% of EtOAc was used.

5,10,15,20,25-pentaphenyl-pentaphyrin (1.1.1.1.1) **12** thus obtained was then characterized by ¹H -NMR and UV-Vis, with the following results.
¹H NMR (200 MHz, CDCl₃, TFA) δ ppm 10.03-9.97 (dd, 6H, p_{y}), 8.73 (dd, 4H, p_{y}), 8.12-7.92 (m, 25H, ph), -3.80 (s, 2H, NH), -4.00 (s, 1H, NH), -5.96 (s, 2H, NH). HPLC-ESI (CH₃CN-H₂O): C₅₅H₃₇N₅ calculated exact mass 767, tR =24.5 min (MH⁺H₂O) = 786, (MH₂²⁺H₂O)/2 = 393.5, MH₂²⁺/2 = 384.5.
UV-Vis (CH2Cl2)= 418nm, 489nm, 527nm, 735nm.

Fully *meso*-substituted (5, 10, 15, 20, 25) R-[1.1.1.1.1] [24] pentaphyrins can be obtained for instance according to method(s) of synthesis analogous to what above described.

While the foregoing is directed to embodiments of the invention, other and further embodiments of the invention may be devised without departing from the basic scope thereof, and the scope thereof is determined by the claims that follow.

### BIBLIOGRAPHY

[1] Stepien, M.; Sprutta, N.; Latos-Grazynski, L. Angew.Chem. Int. Ed. Engl. 2011, 50(19), 4288-4340.
[2] (a) Qian, G.; Wang, Z. Y. Chem. Asian J. 2010, 5, 1006-1029; (b) Sessler, J. L.; Davis, J. M. Acc. Chem. Res. 2001, 34, 989-997.
[3] (a) Ikawa, Y.; Takeda, M.; Suzuki, M.; Osuka, A.; Furuta, H. Chem. Comm., 2010, 46, 5689-5691; (b) Tanaka, T.; Aratani, N.; Lim, J. M.; Kim, K. S.; Kim, D.; A. Osuka Chem. Sci., 2011, 2, 1414-1418; (c) Sarma, T.; Panda, P. K. Chem. Eur. J., 2011, 17, 13987-13991.
[4] (a) Comuzzi, C.; Cogoi, S.; Overhand, M.; Van der Marel, G. A.; Overkleeft, H. S.; Xodo, L. E. J. Med. Chem. 2006, 49, 196-204; (b) Comuzzi, C.; Cogoi, S.; Xodo, L. E. Tetrahedron 2006, 62, 8147-8151.; (c) Ballico, M.; Rapozzi, V.; Xodo, L. E.; Comuzzi, C. Eur. J. Med. Chem. 2011, 46, 712-720.
[5] (a) Allison, R. R.; Bagnato, V. S.; Sibata, C. H. Future Oncol. 2010, 6, 929-940; (b) Nagy, E. M.; Dalla Via, L.; Ronconi, L.; Fregona, D. Curr. Pharm. Des. 2010, 16, 1863-1976; (c) Garg, A. D.; Nowis, D.; Golab, J.; Agostinis, P. Apoptosis 2010, 15, 1050-1071; (d) Almeida Issa, M. C.; Manela-Azulay, M. Bras. Dermatol. 2010, 85, 501-511; (e) Gollnick, S. O.; Brackett, C. M. Immunol. Res. 2010, 46, 216-226; (f) Ortel, B.; Shea, C. R.; Calzavara-Pinton, P. Front. Biosci. 2009, 14, 4157-4172; (g) Dolmans, D. E.; Fukumura, D.; Jain, R. K.; Nat. Rev. Cancer 2003, 3, 380-387; (h) Sibata, C. H.; Colussi, V. C.; Oleinick, N. L.; Kinsella, T. J. Expert Opin. Pharmacother 2001, 2, 917-927; (i) Dougherty, T. J. J. Clin. Laser Med. Surg. 2002, 20, 3-7; (j) Rapozzi, V.; Lombardo, C.; Cogoi, S.; Comuzzi, C; Xodo, L. E. ChemMedChem.2008, 3(4), 565-568.
[6] (a) Hijnen, W. A. M.; Beerendonk, E. F.; Medema, G. J. Water Res. 2006, 40, 3-22; (b) Chong, M. N.; Jin, B.; Chow, C. W. K.; Saint, C. Water Res. 2010, 44, 2997-3027; (c) Sunnotel, O.; Verdoold, R.; Dunlop, P. S. M.; Snelling, W. J.; Lowery, C. J.; Dooley, J. S. G.; Moore, J. E.; Byrne, J. A. J. Water Health 2010, 8, 83-91.
[7] Rossi, G.; Comuzzi, C.; Goi D. J. Water Health, 2012, 10, 390-399.
[8] Burrell, A. K.; Hemmi, G.; Lynch, V.; Sessler, J. L. J. Am. Chem. Soc. 1991, 113, 4690-4692.
[9] Danso-Danquah, R. E.; Xie, L. Y.; Dolphin, D. Heterocycles 1995, 41, 2553-2564.
[10] Král, V.; Brucker, E. A.; Hemmi, G.; Sessler, J. L.; Králová, J.; Bose, H. Bioorg. Med. Chem. 1995, 3, 573-578.
[11] Bruckner, C.; Sternberg, E. D.; Boyle, R. W.; Dolphin, D. Chem. Commun. 1997, 1689-1690.
[12] Maes, W.; Vanderhaeghen, J.; Dehaen, W. Chem. Commun. 2005, 2612-2614.
[13] Rexhausen, H.; Gossauer, A. J. Chem. Soc., Chem. Commun. 1983, 275
[14] Shin, J. Y.; Furuta, H.; Yoza, K.; Igarashi, S.; Osuka, A. J. Am. Chem. Soc. 2001, 123, 7190-7191.
[15] Shin, J. Y.; Furuta, H.; Osuka, A. Angew. Chem., Int. Ed. 2001, 40, 619-621.
[16] Alonso M.; Geerlings P.; De Proft F. The Journal of Organic Chemistry 2013 4419-4431
[17] D.T. Gryko, M. Tosior and B. Koszarna J. Porphyrins Phtal. 2003, 7, 239-248.

## Claims

1. Fully *meso*-substituted (5, 10, 15, 20, 25) R-[1.1.1.1.1] [22] pentaphyrins or fully *meso*-substituted (5, 10, 15, 20, 25) R-[1.1.1.1.1] [24] pentaphyrins or their coordination complexes, having structure: 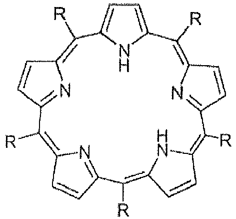 wherein R is a substituent group selected from a group consisting of: penta(4-pyridyl), penta(N-methyl-pyridium-4yl) and penta(phenyl).

2. The use of the pentaphyrins according to claim 1 as biomedical material, or in cation or anion binding applications or protic or neutral molecules binding applications, or as near infrared materials, or as photosensitizers for photodynamic therapy (PDT).

3. A method for the production of fully *meso*-substituted pentaphyrins according to claim 1, the method comprising reacting precursors comprising *meso*-substituted dipyrromethane and *meso*-substituted tripyrromethane or their corresponding phenyl-substituted and adding a suitable catalyst component and an oxidant component.

4. The method of claim 3, wherein the catalyst component is trifluoroacetic acid (TFA).

5. The method of claim 3 or 4, wherein the oxidant component is 2,3-dichloro-5,6-dicyano-1,4-benzoquinone (DDQ).

6. The method of claims 3, 4 or 5, wherein *meso*-(4-pyridyl)dipyrromethane **(13)** having structure: 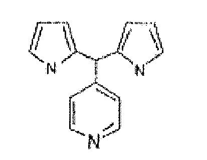 and *meso*-(4-pyridyl)tripyrromethane **(14)** having structure: 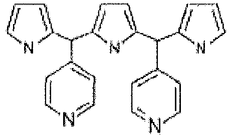 are reacted with pyridinecarboxaldehyde in the presence of the suitable catalyst and with subsequent addition of the oxidant component to obtain 5,10,15,20,25-penta(4-pyridyl)-pentaphyrin (1.1.1.1.1).

7. The method of claim 6, wherein reacting *meso*-(4-pyridyl)dipyrromethane **(13),** *meso*-(4-pyridyl)tripyrromethane **(14)** and pyridinecarboxaldehyde comprises dissolving *meso*-(4-pyridyl)dipyrromethane **(13),** *meso-(4-*pyridyl)tripyrromethane **(14)** and 4-pyridinecarboxaldehyde, adding trifluoroacetic acid (TFA) catalyst to the solution, observing increasingly intense Soret band at 470 nm corresponding to the pentaphyrin and letting the reaction progress until no further gain in Soret band spectral intensity is observed, adding 2,3-dichloro-5,6-dicyano-1,4-benzoquinone (DDQ) oxidant, washing, evaporating and purifying the solution to obtain 5,10,15,20,25-penta(4-pyridyl)-pentaphyrin (1.1.1.1.1).

8. The method of claim 7, further comprising dissolving obtained 5,10,15,20,25-penta(4-pyridyl)-pentaphyrin (1.1.1.1.1), adding excess of methyl iodide, stirring the reactant mixture, evaporating the solvent, washing the solid residue to obtain 5,10,15,20,25-penta(N-methyl-pyridium-4yl)-pentaphyrin (1.1.1.1.1).

9. The method of claims 3, 4 or 5, wherein *meso*-(phenyl)dipyrromethane (15) having structure: 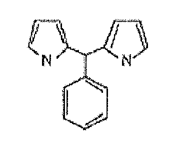 is converted, in the presence of N-benzoylmorpholine and POCl₃, to 1,9-bis(benzoyl)-5-phenyldipyrromethane, then obtained 1,9-bis(benzoyl)-5-phenyldipyrromethane **(17)** having structure: 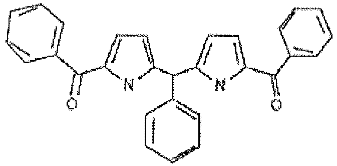 is reacted with NaBH₄ to yield the corresponding phenyldipyrromethane-dicarbinol **(18)** having structure: 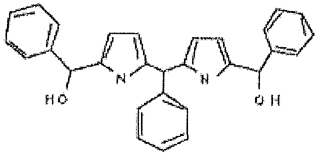 and then *meso*-(phenyl)tripyrromethane **(16)** having structure: 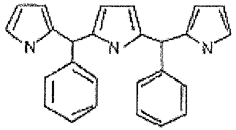 and phenyldipyrromethane-dicarbinol **(18)** are reacted with the catalyst component and addition of the oxidant component to give 5,10,15,20,25-pentaphenyl-pentaphyrin (1.1.1.1.1).

## Patentansprüche

1. Vollständig meso-substituierte (5, 10, 15, 20, 25)-R-[1.1.1.1.1]-[22]-Pentaphyrine oder vollständig meso-substituierte (5, 10, 15, 20, 25)-R-[1.1.1.1.1]-[24]-Pentaphyrine oder ihre Koordinationskomplexe mit der folgenden Struktur: 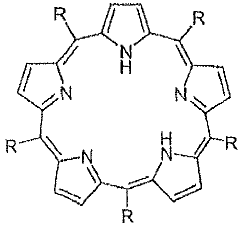 wobei R eine Substituentengruppe ist, die ausgewählt ist aus einer Gruppe bestehend aus: Penta(4-pyridyl), Penta(N-methyl-pyridium-4yl) und Penta(phenyl).

2. Verwendung der Pentaphyrine nach Anspruch 1 als biomedizinisches Material oder in Kation- oder Anion-bindenden Anwendungen oder in protische oder neutrale Moleküle bindenden Anwendungen oder als Nah-Infrarot-Materialien oder als Photosensibilisatoren für die photodynamische Therapie (PDT).

3. Verfahren zur Herstellung von vollständig *meso*-substituierten Pentaphyrinen nach Anspruch 1, wobei das Verfahren das Umsetzen von Vorläufern, umfassend meso-substituiertes Dipyrromethan und meso-substituiertes Tripyrromethan oder ihre entsprechenden Phenyl-substituierten, und die Zugabe einer geeigneten Katalysatorkomponente und einer Oxidationskomponente umfasst.

4. Verfahren nach Anspruch 3, wobei die Katalysatorkomponente Trifluoressigsäure (TFA) ist.

5. Verfahren nach Anspruch 3 oder 4, wobei die Oxidationskomponente 2,3-Dichlor-5,6-dicyano-1,4-benzochinon (DDQ) ist.

6. Verfahren nach Anspruch 3, 4 oder 5, wobei *meso*-(4-Pyridyl)dipyrromethan (13) mit der folgenden Struktur: 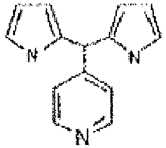 und *meso*-(4-Pyridyl)tripyrromethan (14) mit der folgenden Struktur: 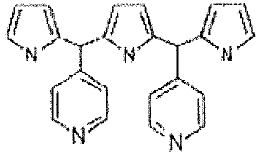 in Gegenwart des geeigneten Katalysators und mit anschließender Zugabe der Oxidationskomponente mit Pyridincarboxaldehyd umgesetzt werden, um 5,10,15,20,25-Penta(4-pyridyl)-pentaphyrin (1.1.1.1.1) zu erhalten.

7. Verfahren nach Anspruch 6, wobei das Umsetzen von *meso*-(4-Pyridyl)dipyrromethan (13), *meso*-(4-Pyridyl)tripyrromethan (14) und Pyridincarboxaldehyd das Auflösen von *meso*-(4-Pyridyl)-dipyrromethan (13), *meso*-(4-Pyridyl)tripyrromethan (14) und 4-Pyridincarboxaldehyd, die Zugabe von Trifluoressigsäure-(TFA)-Katalysator zu der Lösung, das Beobachten der zunehmend intensiven Soret-Bande bei 470 nm entsprechend dem Pentaphyrin und das Zulassen des Fortschreitens der Umsetzung, bis keine weitere Zunahme der Spektralintensität der Soret-Bande beobachtet wird, die Zugabe von 2,3-Dichlor-5,6-dicyano-1,4-benzochinon (DDQ)-Oxidationsmittel, das Waschen, Verdampfen und Reinigen der Lösung, um 5,10,15,20,25-Penta(4-pyridyl)-pentaphyrin (1.1.1.1.1) zu erhalten, umfasst.

8. Verfahren nach Anspruch 7, ferner umfassend das Auflösen des erhaltenen 5,10,15,20,25-Penta(4-pyridyl)-pentaphyrins (1.1.1.1.1), die Zugabe von überschüssigem Methyliodid, das Rühren des Reaktandengemischs, das Verdampfen des Lösungsmittels, das Waschen des festen Rückstands, um 5,10,15,20,25-Penta(N-methyl-pyridium-4yl)-pentaphyrin (1.1.1.1.1) zu erhalten.

9. Verfahren nach Anspruch 3, 4 oder 5, wobei *meso*-(Phenyl)-dipyrromethan (15) mit der folgenden Struktur: 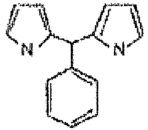 in Gegenwart von N-Benzoylmorpholin und POCl₃ zu 1,9-bis(Benzoyl)-5-phenyldipyrromethan umgewandelt wird, dann das erhaltene 1,9-bis(Benzoyl)-5-phenyldipyrromethan (17) mit der folgenden Struktur: 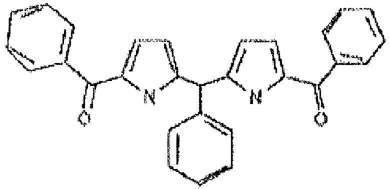 mit NaBH₄ umgesetzt wird, um das entsprechende Phenyldipyrromethan-dicarbinol (18) mit der folgenden Struktur: 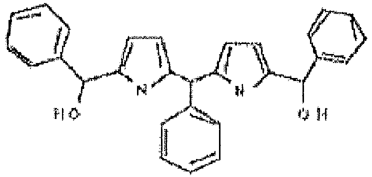 zu ergeben, und dann *meso*-(Phenyl)tripyrromethan (16) mit der folgenden Struktur: 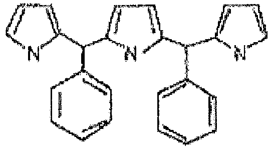 und Phenyldipyrromethan-dicarbinol (18) mit der Katalysatorkomponente unter Zugabe der Oxidationskomponente umgesetzt werden, um 5,10,15,20,25-Pentaphenyl-pentaphyrin (1.1.1.1.1) zu erhalten.

## Revendications

1. (5, 10, 15, 20, 25) R-[1.1.1.1.1] [22]-pentaphyrines totalement *méso*-substituées ou (5, 10, 15, 20, 25) R-[1.1.1.1.1] [24]-pentaphyrines totalement *méso*-substituées ou leurs complexes de coordination, ayant la structure suivante : 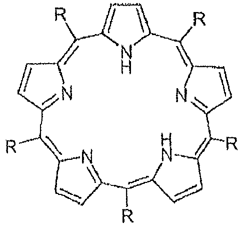 dans laquelle R est un groupe substituant choisi dans le groupe constitué par le penta(4-pyridyl), le penta(N-méthyl-pyridium-4-yl) et le penta(phényl).

2. L'utilisation de pentaphyrines selon la revendication 1 comme matériau biomédical, ou dans des applications impliquant une liaison avec un cation ou un anion ou des applications impliquant une liaison avec des molécules protiques ou neutres ou comme matériaux proches de l'infrarouge ou comme photo-sensibilisateurs pour une thérapie photodynamique (PDT).

3. Un procédé de production de pentaphyrines totalement *méso*-substituées selon la revendication 1, ce procédé comprenant la réaction de précurseurs comprenant du dipyrrométhane *méso*-substitué et du tripyrrométhane méso-substitué ou leur correspondant phényl-substitué et l'addition d'un composé catalyseur approprié et d'un composé oxydant.

4. Le procédé selon la revendication 3, dans lequel le composé catalyseur est l'acide trifluoroacétique (TFA).

5. Le procédé selon la revendication 3 ou 4, dans lequel le composé oxydant est le 2,3-dichloro-5,6-dicyano-1,4-benzoquinone (DDQ).

6. Le procédé selon les revendications 3, 4 ou 5, dans lequel du *méso*-(4-pyridyl)dipyrrométhane (13) ayant la structure 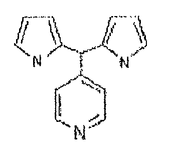 et du *méso*-(4-pyridyl)tripyrrométhane (14) ayant la structure : 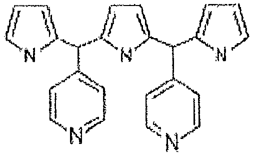 sont mis à réagir avec du pyridine-carboxaldéhyde en présence d'un catalyseur approprié et avec l'addition subséquente du composé oxydant pour obtenir de la 5,10,15,20,25-penta(4-pyridyl)-pentaphyrine (1.1.1.1.1).

7. Le procédé selon la revendication 6, dans lequel la réaction du *méso-*(4-pyridyl)dipyrrométhane (13), du *méso*-(4-pyridyl)tripyrrométhane (14) et du pyridine-carboxaldéhyde comprend la dissolution du *méso*-(4-pyridyl)dipyrrométhane (13), du *méso-*(4-pyridyl)tripyrrométhane (14) et de 4-pyridine-carboxaldéhyde, l'addition d'acide trifluoroacétique (TFA) comme catalyseur à la solution, l'observation d'une bande Soret de plus en plus intense à 470 nm correspondant à la pentaphyrine et le fait de laisser la réaction progresser jusqu'à qu'il n'y ait plus d'augmentation de la bande spectrale Soret, l'addition de l'oxydant 2,3-dichloro-5,6-dicyano-1,4-benzoquinone (DDQ), le lavage, l'évaporation et la purification de la solution pour obtenir de la 5,10,15,20,25-penta(4-pyridyl)-pentaphyrine (1.1.1.1.1).

8. Le procédé selon la revendication 7, comprenant en outre la dissolution de la 5,10,15,20,25-penta(4-pyridyl)-pentaphyrine (1.1.1.1.1) obtenue, l'ajout d'un excès d'iodure de méthyle, l'agitation du milieu réactionnel, l'évaporation du solvant, le lavage du résidu solide pour obtenir de la 5,10,15,20,25-penta(N-méthyl-pyridium-4-yl)-pentaphyrine (1.1.1.1.1).

9. Le procédé selon les revendications 3, 4 ou 5, dans lequel du *méso-*(phényl)dipyrrométhane (15) ayant la structure 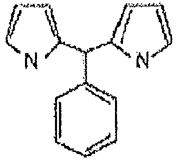 est converti, en présence de N-benzoylmorpholine et de POCl₃, en 1,9-bis(benzoyl)-5-phényldipyrrométhane, puis le 1,9-bis(benzoyl)-5-phényldipyrrométhane (17) ayant la structure : 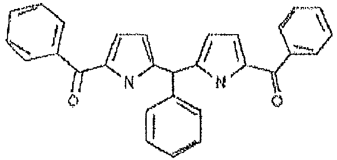 est mis à réagir avec du NaBH₄ pour donner le phényl-dipyrrométhane-dicarbinol (18) correspondant ayant la structure : 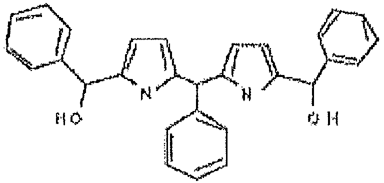 puis le *méso*-(phényl)tripyrrométhane (16) ayant la structure : 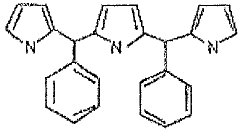 et le phényl-dipyrrométhane-dicarbinol (18) sont mis à réagir avec le composé catalyseur en ajoutant le composé oxydant pour donner le 5,10,15,20,25-pentaphényl-pentaphyrine (1.1.1.1.1).
